# EUROPEAN PATENT SPECIFICATION

(11) **EP 1 374 874 B1**
(45) Date of publication and mention of the grant of the patent: **27.09.2006**
(21) Application number: 03253733.4
(22) Date of filing: 13.06.2003
(51) Int. Cl.: A61K 33/10, A61K 31/047, A61K 47/10, A61K 9/08

(54) **Liquid antacid compositions**
Flüssige Antazida
compositions liquides antiacides

(30) Priority: 14.06.2002 US 171707
(43) Date of publication of application: 02.01.2004
(73) Proprietor: McNEIL-PPC, INC., Skillman, NJ 08858 (US)
(72) Inventor: Hasennayer, Donald L., North Wales, PA 19454 (US); Case, John, Perkasie, PA 18944 (US); Gentry, Abbie, Horsham, PA 19044 (US); Shah, Indu G., North Wales, PA 19454 (US)
(74) Representative: Mercer, Christopher Paul

(56) References cited:
- GB-A- 1 269 987
- US-A- 5 858 413
- DATABASE WPI Section Ch, Week 198828 Derwent Publications Ltd., London, GB; Class A96, AN 1988-194675 XP002253226 & JP 63 132840 A (FUJI KAGAKU KOGYO KK), 4 June 1988 (1988-06-04)

## Description

The present invention relates to liquid antacid preparations comprising at least one acid neutralizing compound and a combination of propylene glycol and glycerin. These preparations have an enhanced resistance to microbial contamination.

### Background of the Invention

Antacid preparations are agents that neutralize or remove acid from the gastric contents. Antacid preparations are widely used in the treatment of various gastrointestinal disorders such as peptic ulcers and gastritis. They are also used for the relief of acid indigestion, heartburn, dyspepsia, sour stomach, reflux esophagitis and the like. The clinical use of antacid preparations is based on their ability to neutralize stomach acid and increase the pH of gastric contents.

Antacid preparations used today are made from a variety of active acid neutralizing compounds such as calcium carbonate, sodium bicarbonate, magnesium salts and aluminum salts. Magnesium hydroxide and aluminum hydroxide are the most potent magnesium and aluminum compounds and are often used in combination. In addition, magnesium oxide, magnesium carbonate, aluminum phosphate, magaldrate and magnesium trisilicate are also employed.

Antacid preparations are typically available as liquid suspensions as well as solid dosage forms. In general, suspensions are preferred to tablets or powders since they are more rapidly and effectively solubilized and have a greater ability to react with and neutralize gastric acid.

Liquid antacid preparations are susceptible to microbial contamination, which generally is controlled by adjustment of the pH of the preparation or adding one or more preservatives. However, it is known that preservatives can degrade in solution depending on the pH. One solution to this problem is to add higher amounts of preservatives. However, this adversely effects the taste of the preparation, since preservatives generally have a bitter taste. This, combined with the poor taste of active acid neutralizing compounds, results in lower patient compliance. Accordingly, there is a need for liquid antacid preparations having both acceptable taste and low susceptibility to microbial contamination over the shelf life of the product.

It is known to add propylene glycol or glycerin to acidic based pharmaceutical elixirs and liquids (with pH's on the order of 3.0 to 5.5). When used in such products, the recommended levels of propylene glycol and glycerin are 15 to 30 weight % and greater than 20 weight %, respectively to function as an antimicrobial preservative. *Handbook of Pharmaceutical Excipients,* 2^{nd} Edition, American Pharmaceutical Association 1994

WO 94/27577 relates to a liquid antacid composition comprising (a) calcium carbonate, (b) short chain alkyl esters of p-hydroxybenzoic acid, (c) benzyl alcohol, (d) optionally but preferably a bis-biguanide compound or a pharmaceutically acceptable salts, esters, isomers, and mixtures thereof, and (e) other excipients. The other excipients may be used to provide an elevated soluble solids content in the composition to enhance preservative efficacy. They may comprise about 60 to about 95 weight % of the composition. Propylene glycol and glycerin are given as examples of the other excipients. However, no amounts of either compound are taught as particularly useful.

GB-A-1269987 discloses an antacid composition which comprises an antacid and at least one pharmaceutically acceptable gelation agent which is a water-dispersible colloidal anionic ether and/or ester of a polymer of a mono-scaccharide, wherein the gelation agent and the antacid are selected and the proportion thereof are such that: (1) the composition contains a major proportion of antacid and a minor proportion of gelation agent based on the combined total weight of the antacid and gelation agent in the composition, (2) the composition, when prepared in aqueous suspension so as to attain a viscosity of 300 cps. at 25°C, must be capable of containing a greater total antacid-solids concentration than that attainable without inclusion of gelation agents, and (3) upon the progressive addition of less than 2 cc. increments of phosphate solution, having the composition defined below, per 100 grams of an aqueous suspension of fluid consistency of the formulation meeting the criteria 1 and 2, an increase in viscosity to a viscosity greater than the initial viscosity of the suspension of fluid consistency is obtained, said phosphate solution prepared by dissolving 41.6 gm of Na₃PO₄, 12H₂O and 55.3gm NaH₂PO₄, H₂O in approximately 25 ml of hot distilled water and diluting the resulting solution to a 100 ml volume.

JP-63132840, as described in DATABASE WPI Section Ch, Week 198828 Derwent Publications Ltd., London, GB; Class A96, AN 1988-194675 XP002253226, discloses an anti-acid formulation that contains (1) 2-20 W/V% of water-insoluble inorganic anti-acid agent which is a metal oxide (e.g. magnesium oxide); a metal hydroxide (e.g. aluminium hydroxide or magnesium hydroxide); a metal carbonate (e.g. calcium carbonate or magnesium carbonate); or a metal compound (e.g. magnesium silicate or aluminate); (2) 0.1-5 W/V% of hydroxyalkylcellulose; and (3) 2-20 W/V% of polybasic alcohol, which is a triol (e.g. glycerine) or a polyol (e.g. sorbitol, mannitol, maltitol or xylitol).

US-A-5858413 discloses a pharmaceutical composition for oral use, comprising: between 12% by weight and 30% by weight of at least one antacid active substance selected from the group consisting of aluminium hydroxide, magnesium hydroxide, magnesium trisilicate, magnesium carbonate, magnesium phosphate, calcium carbonate, calcium phosphate, sodium citrate, magnesium oxide, magaldrate, hydrotalcite, sodium hydrogencarbonate, bismuth subcarbonate, and mixtures thereof; more than 45% by weight of a sugar or sugar alcohol; and between 12% and 35% by weight of water, wherein said composition has a liquid to semisolid consistency and is substantially free of preservatives.

Applicants have now discovered that the addition of specific, low levels of propylene glycol and glycerin to liquid antacid preparations provides excellent preservative efficacy.

Applicants have additionally discovered a liquid antacid preparation with excellent preservative efficacy may be formulated substantially free of preservatives using these specific, low levels of propylene glycol and glycerin.

### Summary of the Invention

According to the present invention there is provided a liquid antacid preparation as defined in the appendant claims.

### Detailed Description of the Invention

Antacids are pharmaceutical products that neutralize at least 5 milliequivalants (mEq) of acid per dose of products. Useful active acid neutralizing compounds are aluminum hydroxide/magnesium hydroxide mixtures. The active acid neutralizing compounds may be utilized as individual powders, e.g. micronized powders, or as amorphous gels. Preferred active acid neutralizing compounds are 13% aluminum hydroxide and magnesium hydroxide 98% in the form of gels. In the aluminum hydroxide 13%/magnesium hydroxide 98% mixtures employed, the weight ratio of aluminum hydroxide 13% to magnesium hydroxide 98% is preferably in the range of about 10:90 to about 90:10, more preferably 50:50.

An antimicrobial adjuvant, selected from mixtures of propylene glycol and glycerin, is present in the preparation in a total amount ranging from about 2 to about 20%. In embodiments where propylene glycol is employed in combination with glycerin, the amount of propylene glycol is preferably from about 3 to about 10%, e.g. from about 4 to about 7%, or in one particular embodiment, the level of propylene glycol is 5%.

Advantageously, these specific, low levels of propylene glycol and glycerin impart excellent preservative efficacy to liquid antacid preparations. This is surprising, in that propylene glycol for example, when present in acidic formulations, is conventionally used at levels of 15 weight % and above to function as an antimicrobial agent. Preservative efficacy of the present preparations is maintained throughout their shelf-life. Preservative efficacy is measured according to <51> Antimicrobial Effectiveness Testing, USP.

In embodiments wherein glycerin is employed in combination with propylene glycol, the level of glycerin is preferably from about 3 to about 10%, e.g. from about 4 to about 7%, or in one embodiment the level of glycerin is 4%. Glycerin in particular has been found to impart good taste to the preparation.

A particularly preferred preparation according to the invention employs about 4 to about 6.25 weight percent propylene glycol and about 3 to about 7 weight percent glycerin. This combination has been found to provide excellent preservative efficacy and taste.

The pH of the preparation is preferably in the range of about 7 to about 12, preferably about 7 to about 11, more preferably about 7 to about 9.

The liquid compositions of the invention are aqueous suspensions containing the active ingredients in admixture with pharmaceutically acceptable excipients typically found in aqueous suspensions for oral administration. Such excipients may be suitable suspending agents, for example, sodium alginate, polyvinylpyrrolidone, gum tragacanth, gum acacia, xanthan gum, locust bean gum and cellulose derivatives such as sodium carboxymethylcellulose, microcrystalline cellulose, hydroxy ethylcellulose, methyl cellulose or hydroxypropyl methylcellulose or mixtures thereof. Also included may be dispersing or wetting agents such as sorbitan esters or lecithin, antigelling additives, surface modifiers, aqueous or non-aqueous vehicles such as sorbitol solution, ethyl alcohol or fractionated vegetable oils, or diluents.

The preparation may also comprise one or more antimicrobial preservatives. The alkyl esters of para-hydroxybenzoic acid (the parabens, e.g. butylparaben, methylparaben and propylparaben) are preferred and may be used alone or in combination. Generally, the parabens are used in a concentration of about 0.02% w/v. Other antimicrobial preservatives include bis-biguanides and sorbic acid.

In one embodiment the preparation may be substantially free of parabens or bis-biguanides, or other conventional antimicrobial preservatives. This embodiment is advantageous in terms of product taste, as the antimicrobial preservatives, in particular parabens, are known to impart an objectionable taste products. It is therefore desirable to use the lowest level of such preservatives required to impart preservative efficacy to the preparation.

The preparation may also contain flavorings, colorants and/or sweeteners as appropriate. Suitable flavorants include fruit flavors, peppermint, licorice or bubble gum flavors. The sweetening agents may be for example bulk sweeteners or polyols (e.g. maltitol, sorbitol) and/or intense sweeteners such as sucralose, saccharin, aspartame or acesulfame K.

Other active agents may be added to the preparation. In one particularly preferred embodiment, an additional active agent is simethicone.

As used herein, the term "simethicone" refers to the broader class of polydimethylsiloxanes, including but not limited to simethicone and dimethicone. Examples of suitable polydimethylsiloxanes, which include, but are not limited to dimethicone and simethicone, are those disclosed in United States Patent Nos. 4,906,478, 5,275,822, and 6,103,260.

The liquid antacid preparation may be made using techniques well known in the pharmaceutical industry. For example, the active acid neutralizing compound, antimicrobial adjuvant and other desired excipients and ingredients may be admixed, dispersed in an aqueous vehicle, and homogenized using equipment and procedures known in the art.

The preparation may be administered, for example 1 to 4 times per day. The dosage will depend on the active acid neutralizing compound employed, the condition being treated, and the age and weight of the patient. Typical dosages include 5-30 mls of the preparation. The dose range for the preparations containing aluminum hydroxide 13% /magnesium hydroxide 98% mixtures is from 200 to 700 mg per 5 ml.

The acid neutralizing capacity of the preparations of the present invention is at least 5 mEq per dose, preferably at least 10 mEq per dose. For typical formulations of the present invention, the acid neutralizing capacity is at least 5 mEq per 20 ml, preferably at least 10 mEq per 20 ml.

In order to further illustrate the present invention and the advantages thereof, the following specific examples are given.

### EXAMPLE 1

Liquid antacid preparations according to the invention were prepared as suspensions containing the following ingredients: aluminum hydroxide USP, magnesium hydroxide USP, propylene glycol USP, propylparaben NF, Glycerine USP, butylparaben NF, sorbitol solution USP 70%, hydroxyethyl cellulose NF, purified water USP, simethicone emulsion USP 30%, sodium saccharin USP, dyes and flavorants. The suspensions were made by first charging and mixing the propylene glycol and glycerin, propylparaben and butylparaben, sorbitol solution, hydroxyethyl cellulose, purified water and simethicone emulsion. Then, the aluminum hydroxide was introduced. Next, the magnesium hydroxide was charged. Sodium saccharin was then added, followed by the dyes. Finally, the flavorants were added, and each batch was completed with a final 30 minute mixing step. The finished batches were each passed through a homogenizer at 500 P.S.I.

Samples from each batch were analyzed for preservative efficacy according to USP 24, <51>. The testing was performed promptly on the initial samples from each batch. All samples met the USP Antimicrobial Effectiveness criteria after 28 days of testing. These samples were formulated with paraben levels adjusted to their expected levels at the end of the product expiration period. It can therefor be concluded that the products will meet USP Antimicrobial Effectiveness criteria at the end of their shelf-life (or expiration period).

| **Example** | **Propylene Glycol (weight %)** | **Glycerin (weight %)** | **Paraben Levels (weight % target)** |
|---|---|---|---|
| 1 | 10 | 10.0 | 25 |
| 2 | 7.5 | 10.0 | 25 |
| 3 | 5.0 | 10.0 | 25 |
| 4 | 10 | 10.0 | 0 |
| 5 | 5.0 | 10.0 | 25 |
| 6 | 10 | 10.0 | 100 |
| 7 | 5.0 | 10.0 | 25 |
| 8 | 5.0 | 5.0 | 25 |
| 9 | 5.0 | 5.0 | 25 |
| 10 | 4.0 | 6.0 | 25 |
| 11 | 3.0 | 7.0 | 25 |
| 12 | 6.25 | 4.0 | 25 |
| 13 | 5.0 | 4.0 | 25 |
| 14 | 5.0 | 3.0 | 25 |
| 15 | 4.0 | 5.0 | 25 |
| 16 | 4.0 | 4.0 | 25 |

The preparations of the invention exhibited excellent preservative efficacy.

### EXAMPLE 2

A liquid antacid preparation according to the invention was compared for taste against commercially available Regular Strength Mylanta® Original Liquid. The preparation according to the invention had the following composition:

| **Ingredient** | **Unit Weight (mg/5ml)** |
|---|---|
| Sorbitol Solution, USP | 953.000 |
| Purified Water USP | 2992.500 |
| Hydroxyethyl Cellulose NF | 17.000 |
| Simethicone Emulsion, USP | 70.000 |
| Magnesium Hydroxide, USP | 204.100 |
| Aluminum Hydroxide, USP | 787.400 |
| Butylparaben NF | 1.000 |
| Propylparaben NF | 1.500 |
| Glycerin, USP | 230.000 |
| Propylene Glycol, USP | 287.500 |
| Sodium Saccharin, USP | 1.000 |
| N&A FF Antacid #19003 | 20.000 |

### Example 3

Using a proto-monadic design, 241 subjects were instructed to swallow a small amount (about 5ml) of one product. They rated four hedonic attributes, three intensity attributes, and three attributes on "Just Right" scales. They were then instructed to taste the second sample. After tasting the second sample, they were instructed to make an overall preference choice. The two products were distributed in a random, balanced order.

The taste of the preparation of the invention was preferred equally to the Regular Strength Mylanta Original Liquid, 49% to 51%.

## Claims

1. A liquid antacid preparation comprising: a) an active acid neutralizing compound consisting of a mixture of aluminum-containing compounds and magnesium-containing compounds; and b) an antimicrobial adjuvant selected from combinations of propylene glycol in an amount from 3 to 10 weight percent with glycerin in an amount from 3 to 10 weight percent based on the total weight of the preparation,
wherein the liquid antacid preparation contains 200 to 700mg of active acid neutralizing compound per 5ml of liquid antacid preparation, and wherein the active acid-neutralizing compound is a mixture of aluminum hydroxide 13% and magnesium hydroxide 98%.

2. The preparation of claim 1, wherein the acid neutralizing capacity of the formulation is at least 5 milli-Equivalents per dose.

3. The preparation of claim 1 wherein the acid neutralizing capacity of the formulation is at least 5 milli-Equivalents per 20 milliliters.

4. The preparation of claim 1 having a pH in the range of 7 to 12.

5. The preparation of claim 1 in suspension form.

6. The preparation of claim 1 further comprising simethicone.

7. The preparation of claim 1 wherein the antimicrobial adjuvant is a combination of propylene glycol and glycerin, and the propylene glycol comprises 4 to 7 weight percent of the preparation.

8. The preparation of claim 1 wherein the antimicrobial adjuvant is a combination of propylene glycol and glycerin, and the glycerin comprises 4 to 7 weight percent of the preparation.

9. A liquid antacid preparation comprising: a) an active acid neutralizing compound consisting essentially of a mixture of aluminum hydroxide and magnesium hydroxide; and b) an antimicrobial adjuvant comprising a combination of propylene glycol in an amount from 3 to 10 weight percent with glycerin in an amount from 3 to 10 weight percent based on the total weight of the preparation
wherein the liquid antacid preparation contains 200 to 700mg of active acid neutralizing compound per 5ml of liquid antacid preparation, and wherein the active acid-neutralizing compound is a mixture of aluminum hydroxide 13% and magnesium hydroxide 98%.

10. The preparation of claim 9 further comprising simethicone.

## Patentansprüche

1. Flüssige Antazidum-Zubereitung, welche umfasst: a) eine aktive säureneutralisierende Verbindung, die aus einer Mischung aluminiumhaltiger Verbindungen und magnesiumhaltiger Verbindungen besteht; und b) ein antimikrobielles Adjuvans, das ausgewählt ist aus Kombinationen von Propylenglykol in einer Menge von 3 bis 10 Gewichtsprozent mit Glycerin in einer Menge von 3 bis 10 Gewichtsprozent, bezogen auf das Gesamtgewicht der Zubereitung,
wobei die flüssige Antazidum-Zubereitung 200 bis 700 mg aktive säureneutralisierende Verbindung pro 5 ml flüssiger Antazidum-Zubereitung enthält und wobei die aktive säureneutralisierende Verbindung eine Mischung aus Aluminiumhydroxid 13% und Magnesiumhydroxid 98% ist.

2. Zubereitung nach Anspruch 1, **dadurch gekennzeichnet, daß** die Säureneutralisierungsfähigkeit der Formulierung wenigstens 5 Milliäquivalente pro Dosis beträgt.

3. Zubereitung nach Anspruch 1, **dadurch gekennzeichnet, daß** die Säureneutralisierungsfähigkeit der Formulierung wenigstens 5 Milliäquivalente pro 20 Milliliter beträgt.

4. Zubereitung nach Anspruch 1, mit einem pH im Bereich von 7 bis 12.

5. Zubereitung nach Anspruch 1, in Suspensionsform.

6. Zubereitung nach Anspruch 1, die weiter Simethicon enthält.

7. Zubereitung nach Anspruch 1, **dadurch gekennzeichnet, daß** das antimikrobielle Adjuvans eine Kombination von Propylenglykol und Glycerin ist und das Propylenglykol 4 bis 7 Gewichtsprozent der Zubereitung umfasst.

8. Zubereitung nach Anspruch 1, **dadurch gekennzeichnet, daß** das antimikrobielle Adjuvans eine Kombination von Propylenglykol und Glycerin ist und das Glycerin 4 bis 7 Gewichtsprozent der Zubereitung umfasst.

9. Flüssige Antazidum-Zubereitung, welche umfasst: a) eine aktive säureneutralisierende Verbindung, die im wesentlichen aus einer Mischung von Aluminiumhydroxid und Magnesiumhydroxid besteht; und b) ein antimikrobielles Adjuvans, das eine Kombination von Propylenglykol in einer Menge von 3 bis 10 Gewichtsprozent mit Glycerin in einer Menge von 3 bis 10 Gewichtsprozent, bezogen auf das Gesamtgewicht der Zubereitung, umfasst,
wobei die flüssige Antazidum-Zubereitung 200 bis 700 mg aktive säureneutralisierende Verbindung pro 5 ml flüssige Antazidum-Zubereitung enthält und wobei die aktive säureneutralisierende Verbindung eine Mischung aus Aluminiumhydroxid 13% und Magnesiumhydroxid 98% ist.

10. Zubereitung nach Anspruch 9, die weiter Simethicon umfasst.

## Revendications

1. Préparation antiacide liquide comprenant : a) un composé actif neutralisant l'acide constitué par un mélange de composés contenant de l'aluminium et de composés contenant du magnésium ; et b) un adjuvant antimicrobien choisi parmi des combinaisons de propylèneglycol en une quantité de 3 à 10 % en poids avec de la glycérine en une quantité de 3 à 10 % en poids par rapport au poids total de la préparation,
dans laquelle la préparation antiacide liquide contient de 200 à 700 mg de composé actif neutralisant l'acide pour 5 mL de préparation antiacide liquide, et dans laquelle le composé actif neutralisant l'acide est un mélange d'hydroxyde d'aluminium à 13 % et d'hydroxyde de magnésium à 98 %.

2. Préparation selon la revendication 1, dans laquelle la capacité à neutraliser l'acide de la formulation est d'au moins 5 milli-équivalents par dose.

3. Préparation selon la revendication 1, dans laquelle la capacité à neutraliser l'acide de la formulation est d'au moins 5 milli-équivalents pour 20 millilitres.

4. Préparation selon la revendication 1, ayant un pH dans la plage de 7 à 12.

5. Préparation selon la revendication 1, sous la forme d'une suspension.

6. Préparation selon la revendication 1, comprenant en outre de la siméthicone.

7. Préparation selon la revendication 1, dans laquelle l'adjuvant antimicrobien est une combinaison de propylèneglycol et de glycérine, et le propylèneglycol représente de 4 à 7 % en poids de la préparation.

8. Préparation selon la revendication 1, dans laquelle l'adjuvant antimicrobien est une combinaison de propylèneglycol et de glycérine, et la glycérine représente de 4 à 7 % en poids de la préparation.

9. Préparation antiacide liquide comprenant : a) un composé actif neutralisant l'acide essentiellement constitué par un mélange d'hydroxyde d'aluminium et d'hydroxyde de magnésium ; et b) un adjuvant antimicrobien comprenant une combinaison de propylèneglycol en une quantité de 3 à 10 % en poids avec de la glycérine en une quantité de 3 à 10 % en poids par rapport au poids total de la préparation,
dans laquelle la préparation antiacide liquide contient de 200 à 700 mg de composé actif neutralisant l'acide pour 5 mL de préparation antiacide liquide, et dans laquelle le composé actif neutralisant l'acide est un mélange d'hydroxyde d'aluminium à 13 % et d'hydroxyde de magnésium à 98 %.

10. Préparation selon la revendication 9, comprenant en outre de la siméthicone.
